# EUROPEAN PATENT SPECIFICATION

(11) **EP 4 178 477 B1**
(45) Date of publication and mention of the grant of the patent: **01.10.2025**
(21) Application number: 21838390.9
(22) Date of filing: 05.07.2021
(51) Int. Cl.: A61B 18/14, A61B 17/00, A61B 18/00

(54) **SYSTEM OF MEDICAL DEVICES FOR PERICARDIAL PUNCTURE**
SYSTEM AUS MEDIZINISCHEN VORRICHTUNGEN ZUR PERIKARDIALEN PUNKTION
SYSTÈME DE DISPOSITIFS MÉDICAUX POUR PERFORATION PÉRICARDIQUE

(30) Priority: 08.07.2020 US 202063049182 P
(43) Date of publication of application: 17.05.2023
(73) Proprietor: Boston Scientific Medical Device Limited, Galway H91 Y868 (IE)
(72) Inventor: MILLER, Brock, Mississauga, Ontario L4W 5P6 (CA); GRAVETT, Matthew, Mississauga, Ontario L4W 5P6 (CA)
(74) Representative: Pfenning, Meinig & Partner mbB
(86) International application number: PCT/IB2021/056017
(87) International publication number: WO 2022/009074

(56) References cited:
- WO-A1-2009/152486
- WO-A1-2014/149472
- WO-A1-2019/215618
- US-A1- 2017 333 698
- US-B2- 10 383 542

## Description

### FIELD:

This document relates to medical devices. More specifically, this document relates to systems of medical devices that can be used in pericardial puncture.

### BACKGROUND

WO-A-2014/149472 discloses a system with an introducer, a puncturing device and an alignment indicator.

### SUMMARY OF THE INVENTION

The invention provides a system of medical devices according to claim 1. Preferred embodiments are defined in the dependent claims. The methods disclosed herein do not form part of the claimed invention.

### SUMMARY OF THE DISCLOSURE

The following summary is intended to introduce the reader to various aspects of the detailed description, but not to define or delimit any invention.

Systems of medical devices are disclosed. According to some aspects, a system of medical devices includes an introducer extending between an introducer proximal end and an introducer distal end. The introducer has a lumen extending therethrough from the introducer proximal end to the introducer distal end. The introducer includes a first indicator electrode that faces radially inwardly toward the lumen. The system further includes a puncture device extending from a puncture device proximal end to a puncture device distal end. The puncture device includes a radiofrequency puncture electrode at the puncture device distal end and a second indicator electrode that is positioned proximal of the radiofrequency puncture electrode and that faces radially outwardly. The puncture device is advanceable through the lumen from the introducer proximal end towards the introducer distal end to position the puncture device in a puncture position in which the radiofrequency puncture electrode is proud of the introducer distal end and in which the second indicator electrode is longitudinally aligned with the first indicator electrode. The system further includes an indicator system that is electrically connectable to the first indicator electrode and the second indicator electrode. The indicator system includes an indicator that is activated when the second indicator electrode is longitudinally aligned with the first indicator electrode.

In some examples, the indicator includes a light, and activation of the indicator includes the illumination of the light.

In some examples, when the second indicator electrode is longitudinally aligned with the first indicator electrode, the second indicator electrode is in electrical contact with the first indicator electrode. Electrical contact of the first indicator electrode and the second indicator electrode can complete an electrical circuit of the indicator system and thereby activate the indicator.

In some examples, the indicator system includes a first impedance sensor for sensing an impedance of the first indicator electrode and a second impedance sensor for sensing an impedance of the second indicator electrode. When the second indicator electrode is in contact with the first indicator electrode, the impedance of the first impedance sensor can approximate the impedance of the second impedance sensor. The indicator can be activated when the impedance of the first impedance sensor approximates the impedance of the second impedance sensor.

The indicator system can be built into the puncture device or the introducer. The puncture device can include a hub at the proximal end, and the indicator can be provided on the hub.

In some examples, the system includes a radiofrequency generator electrically connectable to the radiofrequency puncture electrode. The indicator system and radiofrequency generator can be an all-in-one unit.

Methods for pericardial puncture are also disclosed. According to some aspects, a method for pericardial puncture includes: a. advancing an introducer towards a pericardium; b. advancing a puncture device through the introducer towards the pericardium; c. activating an indicator when an indicator electrode of the introducer is longitudinally aligned with an indicator electrode of the puncture device, to indicate that the puncture device is in a puncture position; and d. after step c., puncturing the pericardium with the puncture device.

In some examples, in the puncture position, a radiofrequency puncture electrode of the puncture device is proud of a distal end of the introducer. Step c. can include delivering radiofrequency energy from a radiofrequency generator to the radiofrequency puncture electrode. The indicator can be part of an all-in-one unit with the radiofrequency generator, or can be built into the puncture device.

In some examples, the indicator includes a light that is illuminated when activated.

In some examples, when the indicator electrode of the introducer is longitudinally aligned with the indicator electrode of the puncture device, the indicator electrode of the introducer is in electrical contact with the indicator electrode of the puncture device. Electrical contact of the indicator electrode of the introducer and the indicator electrode of the puncture device can complete an electrical circuit to activate the indicator.

In some examples, step c. includes sensing an impedance of the indicator electrode of the introducer and sensing an impedance of the indicator electrode of the puncture device. Step c. can include activating the indicator when the impedance of the indicator electrode of the introducer approximates the impedance of the indicator electrode of the puncture device.

### BRIEF DESCRIPTION OF THE DRAWINGS:

The accompanying drawings are for illustrating examples of articles, methods, and apparatuses of the present disclosure and are not intended to be limiting. In the drawings:
Figure 1 is a perspective view of an example system of medical devices;
Figure 2 is a perspective view of the introducer of the system of Figure 1;
Figure 3 is a cross-section taken along line 3-3 in Figure 2;
Figure 4 is a perspective view of the puncture device of the system of Figure 1;
Figure 5 is a cross-section taken along line 5-5 in Figure 4; and
Figure 6 is a cross-section taken along line 6-6 in Figure 1

### DETAILED DESCRIPTION:

Various apparatuses or processes or compositions will be described below to provide an example of an embodiment of the claimed subject matter. No example described below limits any claim and any claim may cover processes or apparatuses or compositions that differ from those described below. The claims are not limited to apparatuses or processes or compositions having all of the features of any one apparatus or process or composition described below or to features common to multiple or all of the apparatuses or processes or compositions described below. It is possible that an apparatus or process or composition described below is not an embodiment of any exclusive right granted by issuance of this patent application. Any subject matter described below and for which an exclusive right is not granted by issuance of this patent application may be the subject matter of another protective instrument, for example, a continuing patent application, and the applicants, inventors or owners do not intend to abandon, disclaim or dedicate to the public any such subject matter by its disclosure in this document.

Generally disclosed herein is a system of medical devices that includes an introducer and a puncture device. The system is configured to provide the user with an indication of the relative position of a radiofrequency puncture electrode of the puncture device and the distal end of the introducer, even if the radio frequency puncture electrode and the distal end of the introducer are not visible with the naked eye. For example, the system can provide the user with an indication that the puncture device is in a puncture position - i.e. positioned with the radiofrequency puncture electrode just proud of the distal end of the introducer, so that radiofrequency energy can be delivered from the radiofrequency puncture electrode to puncture tissue. As will be described in detail below, in order to provide an indication that the puncture device is in the puncture position, the puncture device and introducer can each include a respective indicator electrode, and the indicator electrodes can be longitudinally aligned with each other when the puncture device is in the puncture position. The indicator electrodes can be in communication with an indicator system that, based on signals received from the indicator electrodes, activates an indicator (e.g. a light) when the indicator electrodes are longitudinally aligned.

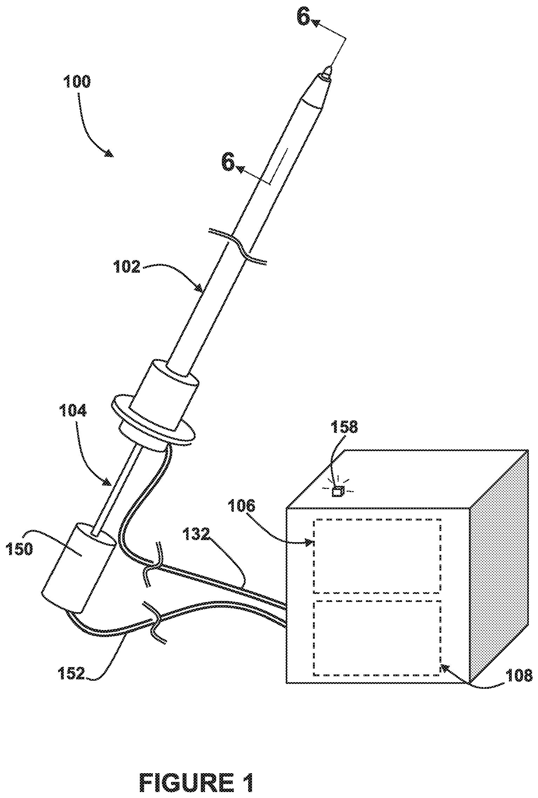

Referring now to Figure 1, an example system 100 of medical devices is shown. The system 100 generally includes an introducer 102 and a puncture device 104, both of which are connected to an indicator system 106. The puncture device 104 is further connected to a radiofrequency (RF) generator 108 (which may in turn be connected to one or more grounding pads, not shown). The puncture device 104 is receivable in and advanceable through the introducer 102. The introducer 102 can serve to atraumatically guide the puncture device 104 towards a target location in a patient's body (e.g. the heart), and the puncture device 104 can then puncture the target location (e.g. puncture the pericardium) using RF energy delivered from the RF generator 108. As will be described in further detail below, the indicator system 106 provides an indication of the position of the puncture device 104 with respect to the introducer 102, in order to facilitate proper positioning of the puncture device 104, enhance ease of use, and enhance patient safety.

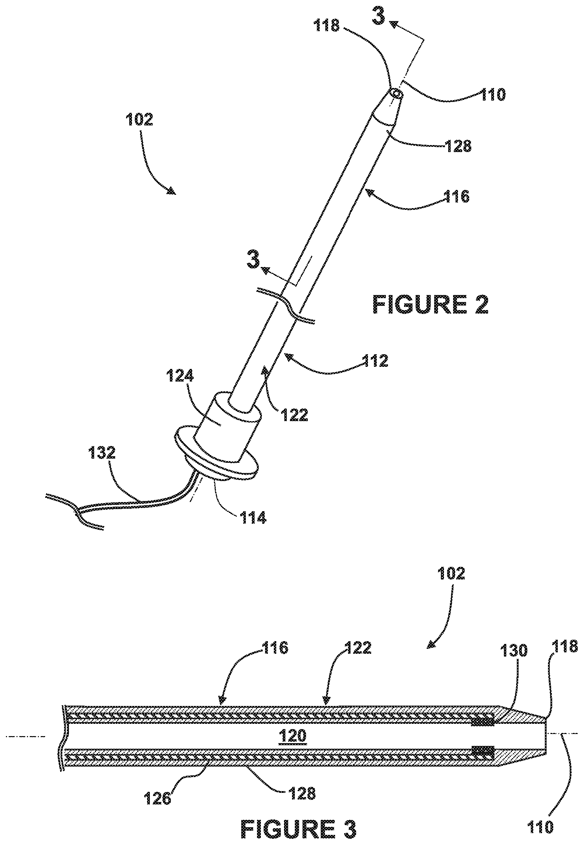

Referring to Figure 2, the introducer 102 extends along a longitudinal axis 110 and has a proximal portion 112 (also referred to herein as an 'introducer proximal portion'), which defines a proximal end 114 (also referred to herein as an 'introducer proximal end'), and a distal portion 116 (also referred to herein as an 'introducer distal portion'), which defines a distal end 118 (also referred to herein as an 'introducer distal end). The introducer 102 has a length (also referred to herein as an 'introducer length') between the proximal end 114 and the distal end 118. A lumen 120 (shown in Figure 3) extends through the introducer 102 from the proximal end 114 to the distal end 118, for receiving the puncture device 104.

Referring still to Figure 2, in the example shown, the introducer 102 includes a shaft 122 (also referred to herein as an 'introducer shaft') and a hub 124 (also referred to herein as an 'introducer hub'). The hub 124 defines the proximal end 114 of the introducer 102, while the shaft 122 defines the distal end 118 of the introducer 102. The hub 124 may include various features, such as fluid ports and hemostatic valves, etc. (not shown). In use, the hub 124 may be grasped and manipulated by the user, while the distal portion 116 is directed to a target site within a patient's body (e.g. the heart). The distal end 118 is blunt, to avoid damaging tissue in contact with the distal end 118.

Referring to Figure 3, the shaft 122 includes a metallic tube 126 (e.g. a stainless steel hypotube), and a polymeric sheathing 128 (e.g. a high-density polyethylene sheathing) on the tube 126. The introducer 102 further includes an indicator electrode 130 (also referred to herein as a 'first indicator electrode'). The indicator electrode 130 faces radially inwardly towards the lumen 120 and is electrically exposed to the lumen 120. In the example shown, the indicator electrode 130 is in the form of a metallic ring that is partially embedded in the polymeric sheathing 128. The metallic tube 126 is electrically connected to the indicator electrode 130, and serves both to provide physical reinforcement for the introducer 102 and as an electrical conductor that electrically connects the indicator electrode 130 to the indicator system 106 (shown in Figure 1). For example, the hub 124 can include electrical connectors (not shown) that allow for the electrical connection of the metallic tube 126 to a cable 132 (shown in Figure 2) that is in turn electrically connected to the indicator system 106. The polymeric sheathing 128 serves to electrically insulate the metallic tube 126.

In alternative examples, the introducer can include a rigid polymer, and the metallic tube can be omitted. A wire can be embedded in the rigid polymer to electrically connect the indicator electrode and the indicator system.

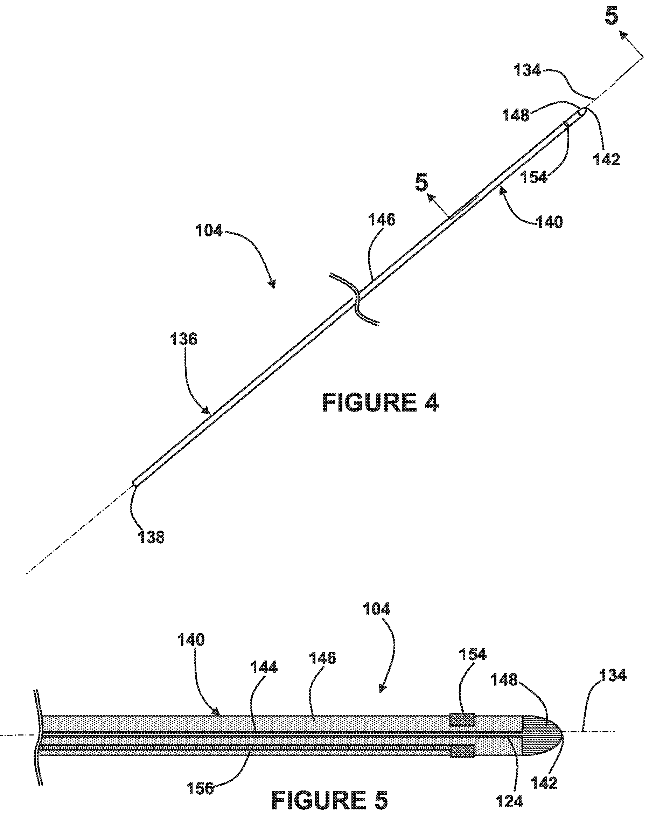

Referring now to Figure 4, the puncture device 104 extends along a longitudinal axis 134 and has a proximal portion 136 (also referred to herein as a 'puncture device proximal portion'), which defines a proximal end 138 (also referred to herein as a 'puncture device proximal end'), and a distal portion 140 (also referred to herein as a 'puncture device distal portion'), which defines a distal end 142 (also referred to herein as a 'puncture device distal end). The puncture device 104 has a length (also referred to herein as a 'length length') between the proximal end 138 and the distal end 142.

Referring to Figure 5, the puncture device 104 is an RF puncture device, and includes a core wire 144 and an electrically insulative material 146 on the core wire 144. An electrically exposed end of the core wire 144 forms an RF puncture electrode 148 at the distal end 142 of the puncture device 104.

Referring to Figure 4, the proximal portion 136 of the puncture device 104 can be used to grasp and manipulate the puncture device 104. Optionally, a guidewire torque device (not shown) can be coupled to the proximal portion 136 to facilitate grasping and manipulation. The proximal portion 136 of the puncture device 104 can be connected to the RF generator 108 via an electrical connector 150 and a cable 152 (both shown in Figure 1) so that RF energy can be delivered from the RF generator 108 to the RF puncture electrode 148 via the core wire 144. When the RF puncture electrode 148 is in contact with a tissue (e.g. the pericardium) and RF energy is delivered to the RF puncture electrode 148, the RF energy causes puncture of the tissue.

In the example shown, the puncture device 104 is relatively flexible, and can also serve as a guidewire.

Referring still to Figure 5, the puncture device 104 further includes its own indicator electrode 154 (also referred to herein as a 'second indicator electrode'). The second indicator electrode 154 is positioned proximally of the RF puncture electrode 148 and faces radially outwardly. In the example shown, the second indicator electrode 154 is in the form of a metallic ring that is partially embedded in the electrically insulative material 146. An electrical conductor 156 in the form of a wire is connected to the second indicator electrode 154, and extends proximally from the second indicator electrode 154 to electrically connect the second indicator electrode 154 to the indicator system 106 (shown in Figure 1). For example, the electrical connector 150 (shown in Figure 1) can allow for the electrical connection of the electrical conductor 156 to the cable 152, which is in turn electrically connected to the indicator system 106 (i.e. in the example shown, the same cable 156 connects the core wire 144 to the RF generator 108 and the electrical conductor 156 to the indicator system 106).

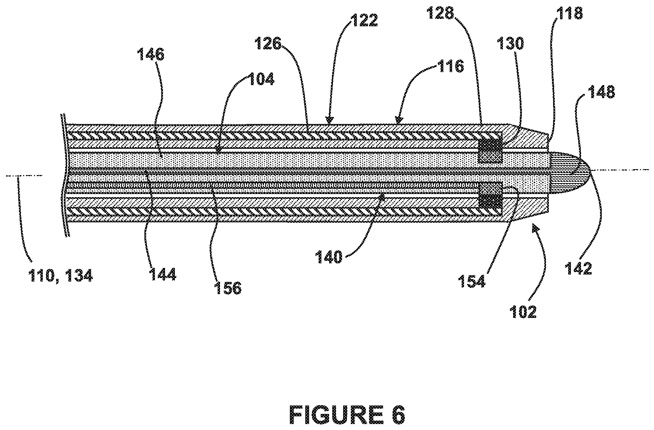

Referring now to Figure 6, the puncture device 104 is advanceable through the lumen 120 (not labelled in Figure 6) of the introducer 102, from the introducer proximal end 114 (not shown in Figure 6) towards the introducer distal end 118, to position the puncture device 104 in a "puncture position". In the puncture position, as shown in Figure 6, the RF puncture electrode 148 is just proud of the introducer distal end 118, so that it can contact and deliver RF energy to a target tissue (e.g. the pericardium), while still being supported by the introducer 102. Furthermore, in the puncture position, the second indicator electrode 154 is longitudinally aligned with and in electrical contact with the first indicator electrode 130.

Referring to Figures 1 and 6, as mentioned above, the indicator system 106 (shown in Figure 1) is electrically connectable to the first indicator electrode 130 and the second indicator electrode 154 (both shown in Figure 6). When the second indicator electrode 154 is in electrical contact with the first indicator electrode 130 - i.e. when the puncture device 104 is in the puncture position - an electrical circuit of the indicator system 106 is completed. This electrical circuit further includes an indicator. In the example shown, the indicator is in the form of a light 158 (shown in Figure 1). When the electrical circuit is completed, the indicator is activated, to indicate to the user that the puncture device 104 is in the puncture position. That is, in the example shown, when the electrical circuit is completed, the light 158 illuminates, to indicate to the user that the puncture device 104 is in the puncture position.

In alternative examples, the indicator can be, for example, a display, and activation of the display can include displaying a certain word or phrase or color. In further alternative examples, the indicator can be a sound-generating apparatus, and activation of the indicator can include the emission of a sound.

In the example shown, the indicator system 106 and radiofrequency generator 108 are part of a single all-in-one unit, and the light 158 is on this unit, as shown in Figure 1. In alternative examples, the indicator system and indicator can be elsewhere, such as built into the puncture device or built into the introducer (e.g.in/on the hub the introducer). In such examples, the indicator system can optionally be battery powered.

In the example shown, the first indicator electrode 130 is in the distal portion 116 of the introducer 102, and the second indicator electrode 154 is in the distal portion 140 of the puncture device 104. In alternative examples, the indicator electrodes 130, 154 can be positioned elsewhere, such as in the proximal portion of the introducer and the proximal portion of the puncture device, respectively.

In an alternative example (not shown), the introducer can include a first indicator electrode and a second indicator electrode, which are spaced apart (e.g. are on opposite sides of the lumen) and are not in direct electrical contact with each other. Each of the indicator electrodes of the introducer can be connected to the indicator system via a respective electrical connector. The puncture device can include a third indicator electrode, and the third indicator electrode can bridge the first and second indicator electrodes when the puncture device is in puncture position, in order to put the first and second indicator electrodes in electrical contact and complete an electrical circuit of the indicator system, and thereby activate an indicator.

In an alternative example (not shown), rather than including an electrical circuit that is completed when the second indicator electrode 154 is in electrical contact with the first indicator electrode 130, the indicator system can include a first impedance sensor for sensing the impedance of the first indicator electrode and a second impedance sensor for sensing the impedance of second electrode, and the indicator system can be configured to activate the indicator when the impedance of the first indicator electrode equals or is similar to (i.e. approximates) the impedance of the second indicator electrode. That is, the impedance sensors can continuously poll the first and second indicator electrodes, respectively. When the puncture device is in the puncture position and the first and second indicator electrodes are in contact, the impedance of the first indicator electrode will be equal to or similar to (i.e. will approximate) the impedance of the second indicator electrode. When the impedance of the first indicator electrode approximates the impedance of the second indicator electrode, the indicator can be activated.

In any of the above examples, the introducer 102 and/or puncture device 104 may include a radiopaque marker (not shown), to facilitate viewing of the introducer 102 and/or puncture device 104 under fluoroscopy.

In one particular example of use, the introducer 102 may be advanced towards the pericardium, to position the introducer distal end 118 proximate the pericardium, and preferably slightly spaced from the pericardium. The introducer 102 may be advanced towards the pericardium percutaneously, via the subxiphoid approach. The introducer 102 may be advanced with a stylet (not shown) received in the lumen 120. The positioning of the introducer 102 may be confirmed using fluoroscopy.

The puncture device 104 may then be advanced through the introducer 102, towards the pericardium, until the puncture device 104 is in the puncture position and in contact with the pericardium. In order to achieve this, the puncture device 104 can be advanced through the introducer 102 until the indicator is activated (i.e. until the light 158 is illuminated). Activation of the indicator indicates to the user that the puncture device 104 is in the puncture position, and occurs when the indicator electrode 130 of the introducer 102 is in electrical contact with the indicator electrode 154 of the puncture device 104, which in turn occurs when the indicator electrode 130 of the introducer 102 is longitudinally aligned with the indicator electrode 154 of the puncture device 104. which in turn occurs when the puncture device 104 is in the puncture position.

Optionally, fluoroscopy can be used to confirm that the puncture device 104 is in the puncture position.

Once it has been confirmed that the puncture device 104 is in the puncture position, RF energy can be delivered to the RF puncture electrode 148 from the RF generator 108, and from the RF puncture electrode 148 to puncture the pericardium.

Once the target tissue has been punctured, the puncture device 104 can be advanced into the pericardial space, and can serve as a guidewire in subsequent steps of the medical procedure.

While the above description provides examples of one or more processes or apparatuses or compositions, it will be appreciated that other processes or apparatuses or compositions may be within the scope of the accompanying claims.

## Claims

1. A system (100) of medical devices, comprising:
an introducer (102) extending between an introducer proximal end (114) and an introducer distal end (118), wherein the introducer has a lumen (120) extending therethrough from the introducer proximal end to the introducer distal end, and wherein the introducer comprises a first indicator electrode (130) that faces radially inwardly toward the lumen; a puncture device (104) extending from a puncture device proximal end (138) to a puncture device distal end (142),
wherein the puncture device comprises a radiofrequency puncture electrode (148) at the puncture device distal end and a second indicator electrode (154) that is positioned proximal of the radiofrequency puncture electrode and that faces radially outwardly, wherein the puncture device is advanceable through the lumen from the introducer proximal end towards the introducer distal end to position the puncture device in a puncture position in which the radiofrequency puncture electrode is proud of the introducer distal end and in which the second indicator electrode is longitudinally aligned with the first indicator electrode;
an indicator system (106) electrically connectable to the first indicator electrode and the second indicator electrode and comprising an indicator that is activated when the second indicator electrode is longitudinally aligned with the first indicator electrode.

2. The system of claim 1, wherein the indicator comprises a light (158), and activation of the indicator comprises the illumination of the light.

3. The system of claim 1, wherein when the second indicator electrode is longitudinally aligned with the first indicator electrode, the second indicator electrode is in electrical contact with the first indicator electrode.

4. The system of claim 3, wherein electrical contact of the first indicator electrode and the second indicator electrode completes an electrical circuit of the indicator system and thereby activates the indicator.

5. The system of claim 3, wherein the indicator system comprises a first impedance sensor for sensing an impedance of the first indicator electrode and a second impedance sensor for sensing an impedance of the second indicator electrode.

6. The system of claim 5, wherein when the second indicator electrode is in contact with the first indicator electrode, the impedance of the first impedance sensor approximates the impedance of the second impedance sensor.

7. The system of claim 6, wherein the indicator is activated when the impedance of the first impedance sensor approximates the impedance of the second impedance sensor.

8. The system of claim 1, wherein the indicator system is built into the puncture device or the introducer.

9. The system of claim 8, wherein the introducer comprises a hub (124) at the introducer proximal end, and the indicator is provided on the hub.

10. The system of claim 1, further comprising a radiofrequency generator (108) electrically connectable to the radiofrequency puncture electrode, wherein the indicator system and radiofrequency generator are an all-in-one unit.

## Patentansprüche

1. System (100) aus medizinischen Vorrichtungen, umfassend:
eine Einführungsvorrichtung (102), die sich zwischen einem proximalen Ende (114) der Einführungsvorrichtung und einem distalen Ende (118) der Einführungsvorrichtung erstreckt, wobei die Einführungsvorrichtung ein Lumen (120) aufweist, das sich dadurch von dem proximalen Ende der Einführungsvorrichtung zu dem distalen Ende der Einführungsvorrichtung erstreckt, und wobei die Einführungsvorrichtung eine erste Indikatorelektrode (130) umfasst, die radial nach innen zu dem Lumen gewandt ist;
eine Punktionsvorrichtung (104), die sich von einem proximalen Ende (138) der Punktionsvorrichtung zu einem distalen Ende (142) der Punktionsvorrichtung erstreckt,
wobei die Punktionsvorrichtung eine Radiofrequenz-Punktionselektrode (148) an dem distalen Ende der Punktionsvorrichtung und eine zweite Indikatorelektrode (154), die proximal zu der Radiofrequenz-Punktionselektrode positioniert ist und radial nach außen gewandt ist, umfasst, wobei die Punktionsvorrichtung durch das Lumen von dem proximalen Ende der Einführungsvorrichtung zu dem distalen Ende der Einführungsvorrichtung vorbewegbar ist, um die Punktionsvorrichtung in einer Punktionsposition zu positionieren, in der die Radiofrequenz-Punktionselektrode von dem distalen Ende der Einführungsvorrichtung hervorsteht und in der die zweite Indikatorelektrode longitudinal zu der ersten Indikatorelektrode ausgerichtet ist;
ein Indikatorsystem (106), das elektrisch mit der ersten Indikatorelektrode und der zweiten Indikatorelektrode verbindbar ist und einen Indikator umfasst, der aktiviert wird, wenn die zweite Indikatorelektrode longitudinal zu der ersten Indikatorelektrode ausgerichtet ist.

2. System nach Anspruch 1, wobei der Indikator eine Leuchte (158) umfasst und eine Aktivierung des Indikators Leuchtenlassen der Leuchte umfasst.

3. System nach Anspruch 1, wobei, wenn die zweite Indikatorelektrode longitudinal zu der ersten Indikatorelektrode ausgerichtet ist, die zweite Indikatorelektrode in elektrischem Kontakt mit der ersten Indikatorelektrode steht.

4. System nach Anspruch 3, wobei der elektrische Kontakt der ersten Indikatorelektrode und der zweiten Indikatorelektrode einen Stromkreis des Indikatorsystems schließt und dadurch den Indikator aktiviert.

5. System nach Anspruch 3, wobei das Indikatorsystem einen ersten Impedanzsensor zum Erfassen einer Impedanz der ersten Indikatorelektrode und einen zweiten Impedanzsensor zum Erfassen einer Impedanz der zweiten Indikatorelektrode umfasst.

6. System nach Anspruch 5, wobei, wenn die zweite Indikatorelektrode mit der ersten Indikatorelektrode in Kontakt steht, sich die Impedanz des ersten Impedanzsensors an die Impedanz des zweiten Impedanzsensors annähert.

7. System nach Anspruch 6, wobei der Indikator aktiviert wird, wenn sich die Impedanz des ersten Impedanzsensors an die Impedanz des zweiten Impedanzsensors annähert.

8. System nach Anspruch 1, wobei das Indikatorsystem in die Punktionsvorrichtung oder die Einführungsvorrichtung eingebaut ist.

9. System nach Anspruch 8, wobei die Einführungsvorrichtung eine Schleuse (124) an dem proximalen Ende der Einführungsvorrichtung umfasst und der Indikator an der Schleuse bereitgestellt ist.

10. System nach Anspruch 1, ferner umfassend einen Radiofrequenzgenerator (108), der elektrisch mit der Radiofrequenz-Punktionselektrode verbindbar ist, wobei das Indikatorsystem und der Radiofrequenzgenerator eine All-in-One-Einheit sind.

## Revendications

1. Système (100) de dispositifs médicaux, comprenant :
un introducteur (102) qui est étendu entre une extrémité proximale d'introducteur (114) et une extrémité distale d'introducteur (118), dans lequel l'introducteur comporte une lumière (120) qui est étendue au travers depuis l'extrémité proximale d'introducteur jusqu'à l'extrémité distale d'introducteur, et dans lequel l'introducteur comprend une première électrode d'indicateur (130) qui est orientée radialement vers l'intérieur en direction de la lumière ;
un dispositif de perforation (104) qui est étendu depuis une extrémité proximale de dispositif de perforation (138) jusqu'à une extrémité distale de dispositif de perforation (142),
dans lequel le dispositif de perforation comprend une électrode de perforation radiofréquences (148) au niveau de l'extrémité distale de dispositif de perforation et une seconde électrode d'indicateur (154) qui a une position proximale de l'électrode de perforation radiofréquences et qui est orientée radialement vers l'extérieur, dans lequel le dispositif de perforation peut être avancé au travers de la lumière depuis l'extrémité proximale d'introducteur en direction de l'extrémité distale d'introducteur afin de positionner le dispositif de perforation selon une position de perforation dans laquelle l'électrode de perforation radiofréquences fait saillie de l'extrémité distale d'introducteur et dans laquelle la seconde électrode d'indicateur est alignée longitudinalement avec la première électrode d'indicateur ; et
un système à indicateur (106) qui peut être connecté électriquement à la première électrode d'indicateur et à la seconde électrode d'indicateur et qui comprend un indicateur qui est activé lorsque la seconde électrode d'indicateur est alignée longitudinalement avec la première électrode d'indicateur.

2. Système selon la revendication 1, dans lequel l'indicateur comprend un moyen d'éclairage (158) et l'activation de l'indicateur comprend l'éclairage du moyen d'éclairage.

3. Système selon la revendication 1, dans lequel, lorsque la seconde électrode d'indicateur est alignée longitudinalement avec la première électrode d'indicateur, la seconde électrode d'indicateur est en contact électrique avec la première électrode d'indicateur.

4. Système selon la revendication 3, dans lequel le contact électrique de la première électrode d'indicateur et de la seconde électrode d'indicateur parachève un circuit électrique du système à indicateur et active de ce fait l'indicateur.

5. Système selon la revendication 3, dans lequel le système à indicateur comprend un premier capteur d'impédance pour détecter une impédance de la première électrode d'indicateur et un second capteur d'impédance pour détecter une impédance de la seconde électrode d'indicateur.

6. Système selon la revendication 5, dans lequel, lorsque la seconde électrode d'indicateur est en contact avec la première électrode d'indicateur, l'impédance du premier capteur d'impédance est proche de l'impédance du second capteur d'impédance.

7. Système selon la revendication 6, dans lequel l'indicateur est activé lorsque l'impédance du premier capteur d'impédance est proche de l'impédance du second capteur d'impédance.

8. Système selon la revendication 1, dans lequel le système à indicateur est intégré à l'intérieur du dispositif de perforation ou de l'introducteur.

9. Système selon la revendication 8, dans lequel l'introducteur comprend un moyeu (124) au niveau de l'extrémité proximale d'introducteur, et l'indicateur est prévu sur le moyeu.

10. Système selon la revendication 1, comprenant en outre un générateur de radiofréquences (108) qui peut être connecté électriquement à l'électrode de perforation radiofréquences, dans lequel le système à indicateur et le générateur de radiofréquences sont une unité tout en un.
